# EUROPÄISCHE PATENTSCHRIFT

(11) **EP 1 951 195 B1**
(45) Veröffentlichungstag und Bekanntmachung des Hinweises auf die Patenterteilung: **24.12.2014**
(21) Anmeldenummer: 06829014.7
(22) Anmeldetag: 13.11.2006
(51) Int. Cl.: A61K 8/97, A61Q 19/10, C11D 3/382, C09K 3/14, A61K 8/73, C11D 3/14

(54) **VERFAHREN ZUR HERSTELLUNG EINES KOSMETISCHEN ABRASIVUMS**
METHOD OF PRODUCING A COSMETIC ABRASIVE
PROCEDE DE FABRICATION D'UN ABRASIF COSMETIQUE

(30) Priorität: 16.11.2005 DE 102005054976
(43) Veröffentlichungstag der Anmeldung: 06.08.2008
(73) Patentinhaber: Deb IP Limited, Denby, Derbyshire DE5 8JZ (GB)
(72) Erfinder: DANIEL, Günther, 47799 Krefeld (DE); FRIEBEL, Michael, 47918 Tönisvorst (DE)
(74) Vertreter: Elsy, David
(86) Internationale Anmeldenummer: PCT/EP2006/010845
(87) Internationale Veröffentlichungsnummer: WO 2007/057134

(56) Entgegenhaltungen:
- EP-A2- 1 106 173
- WO-A-92/09265
- WO-A-2004/071483
- DE-A1- 10 329 933

## Beschreibung

Die vorliegende Erfindung betrifft ein Verfahren zur Herstellung eines kosmetischen Abrasivums bzw. dessen Verwendung in kosmetischen Produkten.

Ein wesentlicher Bestandteil kosmetischer Reinigungs- und Behandlungsmittel ist das Reibemittel, das die Aufgabe hat, die Reinigungswirkung von waschaktiven bzw. tensidartigen Komponenten mechanisch zu unterstützen.

Im Stand der Technik sind zahlreiche anorganische und organische Materialien beschrieben, die sich in Reinigungspräparaten als mechanische Reinigungs- und Behandlungsmittel einsetzen lassen, insbesondere in Handreinigern oder in sogenannten Peeling-Cremes und Spezialreinigungs-Gelen. Sie dienen hierbei zur Entfernung der oberen, abgestorbenen Hautzellen oder von Hautverunreinigungen, beispielsweise im Gesicht oder an anderen Körperteilen.

Besonderes Interesse haben hierbei natürliche Abrasivstoffe, wie z.B. gewaschene und gemahlene Schalen von Walnüssen sowie gemahlene Aprikosenkerne oder Olivenkerne gefunden, die aufgrund ihrer Härte und Korngröße für die oberflächliche Hautreinigung geeignet sind. Solche natürlichen Abrasivstoffe haben eine schonende, sehr gut reinigende Wirkung, ohne die Haut zu zerkratzen. Nachteilig an solchen natürlichen Abrasivstoffen ist, daß sie zu kosmetischen Produkten führen, die ein dunkles, schmutziges Aussehen haben. Um dies zu verhindern, sind diesen kosmetischen Produkten aufhellende Pigmente wie beispielsweise Titandioxid zuzusetzen.

In der EP 0 559 696 B1 wird ein Verfahren zur Herstellung von mit einem Bleichmittel, insbesondere Wasserstoffperoxid behandeltem Material in feiner Verteilung aus natürlichen Schalen und/oder Kernen beschrieben bzw. die Verwendung des so gewonnenen gebleichten Abrasivums in kosmetischen Produkten dargestellt.

Das in dieser Patentschrift beschriebene Verfahren zur Bleichung von Naturmehlen, das Mehle bestimmter Korngröße, vorzugsweise Walnussschalenmehl, bleicht und trocknet, wird seit vielen Jahren im großtechnischen Maßstab erfolgreich angewandt. Die hiernach hergestellten Mehle haben üblicherweise eine Keimzahl kleiner als 10² KBE/g und sind frei von pathogenen Keimen, weisen eine hellbeige Farbe auf und werden in Handwaschpasten zur industriellen Handreinigung eingesetzt. Hierbei hat sich der durch die Mehle hervorgerufene abrasive Effekt zur physikalischen Entfernung von Industrieschmutzen seit vielen Jahren als sehr adäquat und effektiv erwiesen.

Da das Bleichen der natürlichen Kern- und Schalenmehle in wasserhaltiger Suspension durchzuführen ist, fallen auch große Mengen an Ablaugen an, wie die umfangreiche Praxis dieses Verfahrens gezeigt hat. Darüber hinaus werden in dem Verfahren auch Stabilisierungs- und Reduktionsmittel eingesetzt, die natürlich als zusätzliche Rohstoffe dieses Verfahren verteuern im Vergleich zu einem möglichen Verfahren, in dem auf solche Stabilisierungs- und Reduktionsmittel verzichtet werden könnte.

Zur Vermeidung solcher Ablaugen wird in der EP 1 136 063 A2 ein Bleichverfahren vorgeschlagen, in dem in einem "trockenen" Prozess Persäuren auf das natürliche Mehl, das als biologisches Material aus einer Vielzahl pflanzlicher Materialien gewonnen werden kann, aufgesprüht werden und eine Aufhellung bewirken sollen. Die Mischung dieses biologischen Materials mit dem Bleichmittel soll höchstens 60 Gew.-% Wasser enthalten, wobei nach dem Mischungsvorgang ein Nachreifungsprozeß einsetzt. Nach 10 Tagen soll das während des Verfahrens gebildete Peroxid im erhaltenen Produkt nicht mehr nachweisbar sein.

Nachteilig an einem solchen Verfahren ist allerdings, daß Restmengen an Carbonsäuren im fertigen Mehl enthalten sind, die durch Bildung von Salzfrachten bei Herstellung der kosmetischen Endprodukte, insbesondere in Handwaschpasten umweltbelastend wirken. Darüber hinaus werden auch in diesem Verfahren bis zu 5 Gew.-%, bezogen auf die das biologische Material und das Bleichmittel umfassende Mischung, Stabilisierungsmittel in Form von Moderatoren eingesetzt. Entsprechendes gilt für den Einsatz von Reduktionsmitteln zum Zerstören von überschüssigen Peroxiden.

In der DE 103 05 959 wird ein Verfahren beschrieben, dessen Bleichergebnis im Hinblick auf Keimgehalt, Geruch und Farbe nicht nur zu Abrasiva führt, die in ihrem Eigenschaftsprofil vergleichbar bzw. besser sind als die nach dem in der EP 0 559 696 B1 beschriebenen Verfahren erhaltenen Abrasiva, die bei ihrem Einsatz in kosmetischen Reinigungsmitteln bei geringstmöglicher Konzentration bzw. unter völligem Verzicht an aufhellenden Substanzen optisch helle und kosmetisch akzeptable Produkte liefern. Weiterhin wird in diesem Verfahren auch vollständig auf den Einsatz von Stabilisatoren und Reduktionsmitteln im Produktionsprozeß verzichtet. Die nach dem Waschprozeß der mikrobiologisch dekontaminierten, deodorierten und gebleichten Mehle anfallenden Abwässer sind hinsichtlich ihrer biologischen Abbaubarkeit verbessert und auch der gesamte Produktionsprozeß ist in betriebswirtschaftlicher Hinsicht kostengünstiger gestaltet.

In dem vorgenannten Verfahren werden natürliche Kerne, Schalen, Fruchthülsen und/oder Samen zu einem Mehl definierter Korngröße gemahlen. Anschließend wird das erhaltene Mehl in wässriger Suspension mit 1,0 bis 10,0 Gew.-% eines Bleichmittels, bezogen auf die gesamte Ansatzmenge, behandelt. Hierbei erfolgt die Zugabe des Bleichmittels in zwei Schritten, wobei im ersten Schritt nach Zugabe von 20 bis 40 Gew.-% des Bleichmittels, bezogen auf die gesamte Einsatzmenge des Bleichmittels, ein pH-Wert-Bereich von 3 bis 5 erhalten wird.

Nichtsdestoweniger stellte sich bei diesem Zweistufenprozeß im Produktionsbetrieb als nachteilig heraus, daß es durch die Art und Menge der Bleichmitteldosierung, insbesondere der Wasserstoffperoxid-Dosierung es chargenabhängig zu erhöhten Keimzahlen und sehr differenzierter Aufhellung kommen konnte:
Die Nichterreichung der geforderten maximalen Keimzahl von 10² KBE/g in diesen Chargen - wobei durchaus Keimzahlen im Bereich von 10³ KBE/g gemessen wurden-führte dazu, daß immer wieder abgefüllte Ware aus big bags erneut in den Bleichprozeß zurückgeführt werden mußte. So waren ca. 15% der gesamten Menge des zu bleichenden Mehls als verkeimte Ware wieder in den Prozeß zurückzuführen.

Dies hatte zur Folge, daß die durchzuführenden logistischen Schritte wie Lagerhaltung, Transport und Mischungsprozesse von dem Ergebnis der Keimzahlbestimmung abhängig waren und daher so geplant werden mußten, um der unabdingbaren Zuführung dieser verkeimten Mehle in einem weiteren Bleichprozeß Rechnung zu tragen. Die Mehle wurden durch diese zweite Behandlung in einen adäquaten mikrobiologischen Zustand versetzt, so daß sie dann ihrer bestimmungsgemäßen Verwendung als natürliche Abrasiva in kosmetischen Produkten zugeführt werden konnten.

Der durch dieses Verfahren neben der Entkeimung bewirkte Aufhellungsprozeß erwies sich im kontinuierlichen Produktionsbetrieb mitunter ebenfalls als instabil, da es hin und wieder zu deutlich sichtbaren Unterschieden in der Endfarbe des behandelten Mehls kam, die nicht nur durch die Bandbreite der Ausgangsfarbe im Rohstoff begründet war. So traten auch Chargen mit einer für die Verwendung in Kosmetika nicht tolerierbaren dunkleren Farbe auf.

Diese zusätzlichen arbeitsintensiven Prozeßschritte, insbesondere die damit verbundenen Ausbeuteverluste führen letztendlich zu erheblichen Mehrkosten des gesamten Herstellungsprozesses.

Darüberhinaus ist erkannt worden, daß neben der Entkeimung und der Helligkeit der durch das Verfahren hergestellten Abrasiva auch deren Fettgehalt zu berücksichtigen ist, da der Fettgehalt der Abrasiva einen nicht zu vernachlässigenden Einfluß auf die Eigenschaften, insbesondere die Viskosität der unter Verwendung der gebleichten Abrasiva hergestellten Kosmetika, vorzugsweise Haut- und Handreinigungsmittel wie z.B. Grobhandreiniger, hat.

Es besteht somit immer noch ein Bedarf an einem Herstellungsverfahren für Abrasiva, die für kosmetische Erzeugnisse bestimmt sind, das eine umweltschonende und wirtschaftliche Gestaltung des Produktionsprozesses, der zur mikrobiologischen Dekontaminierung, Deodorierung und Bleichung und auch Entfettung von natürlichen Kern- und Schalenmehlen führt, gewährleistet.

Aufgabe war es daher, ein Verfahren zur Bleichung von natürlichen Kernen, Schalen, Fruchthülsen und/oder Samen zur Herstellung kosmetischer Abrasiva bereitzustellen, das auch im groß- bzw. produktionstechnischen Maßstab eine Ausschußquote durch Verkeimungen unter 0,5 % und eine sehr gute mikrobiologische Stabilität aufweist, wobei eine gleichmäßige Aufhellung/Farbe bei möglichst geringem Fettgehalt über die gesamte Bleichkampagne sichergestellt ist.

Die obige Aufgabe konnte erfindungsgemäß durch ein Verfahren zur Herstellung eines abrasiven Stoffes gelöst werden, wobei natürliche Kerne, Schalen, Fruchthülsen und/oder Samen zu einem Mehl definierter Korngröße gemahlen werden, das Mehl in wässriger Suspension mit mindestens einem Bleichmittel behandelt wird, wobei die Zugabe der Bleichmittelmenge in zwei Schritten erfolgt, in dem im ersten Schritt wenigstens 40 Gew.-% bis 90 Gew.-% der Bleichmittelgesamtmenge zugegeben und das Mehl im saurem Medium entkeimt wird und im zweiten Schritt die Aufhellung und Entfettung des Mehles durch Simultandosierung der Bleichmittelrestmenge mit einer Alkalienlösung in alkalischem Medium bewirkt wird.

Erfindungsgemäß werden als natürliche Schalen- oder Kernmehle Walnußschalen-mehl, Mandelschalenmehl, Haselnußschalenmehl, Olivenkernmehl, Aprikosenkemmehl, Pfirsichkernmehl, Kirschkernmehl, Pflaumenkernmehl oder sonstiges natürliches Schalen- oder Kernmehl, beispielsweise von Palmkernen und Kokosnüssen, Jojobafrüchten, Macademia-Nüssen und anderen Nüssen, Pistazien- und Pinienschalen und anderes Kernobst sowie ein beliebiges Gemisch der genannten Materialien eingesetzt. Besonders bevorzugt als natürliches Schalen- oder Kernmehl ist erfindungsgemäß Walnußschalenmehl.

Darüber hinaus lassen sich auch weitere im Stand der Technik als milde Abrasiva bekannte Pflanzenmehle aus Fruchthülsen und Samen, wie z.B. Maiskolbenmehl, Weizenkleie, Hafermehl aber auch beliebige Holzmehle mit dem erfindungsgemäßen Verfahren bleichen.

Um eine definierte Korngröße der im erfindungsgemäßen Verfahren einzusetzenden Mehle von natürlichen Kernen, Schalen, Fruchthülsen und/oder Samen zu erhalten, werden diese in an sich bekannter Weise zu einem Mehl, gegebenenfalls unter Einbeziehung einer Klassifizierung durch Siebe gemahlen. Mehle, die eine Korngröße von 50 bis 2000 µm, vorzugsweise von 70 bis 1000 µm und besonders bevorzugt von 80 bis 400 µm besitzen, können im erfindungsgemäßen Verfahren verwendet werden.

Zum Mahlen der Mehle können die im Stand der Technik bekannten Zerkleinerungsapparate bzw. Mühlen eingesetzt werden, wie sie z. B. in der EP 0 559 696 ausgeführt worden sind, insbesondere Feinprallmühlen mit Pendel- oder Plattenschlägerwerk, Passagenwalzwerke, Hammerschlag- oder Stiftmühlen gegebenenfalls mit Klassierungsaggregaten, wie z. B. Condux-Mühlen etc.

Die zu einem Mehl definierter Korngröße gemahlenen natürlichen Kerne, Schalen, Fruchthülsen und/oder Samen werden in wässriger Suspension mit mindestens einem Bleichmittel behandelt. Als Bleichmittel können alle Verbindungen verwendet werden, die eine irreversible Zerstörung der Chromophore in diesen Naturmehlen gewährleisten, wobei die gebleichten Mehle bei der erfindungsgemäßen Bleichbehandlung nicht oder nur unwesentlich chemisch verändert werden, so daß sie als Abrasiva in kosmetischen Produkten Anwendung finden können. Solche Bleichmittel sind z.B. sogenannte oxidierende Bleichmittel, wie sie beispielsweise in Ullmanns Encyklopädie der technischen Chemie, 4. Auflage, Bd. 8, Seiten 589 bis 595 beschrieben sind. Bevorzugt sind anorganische und organische Peroxide wie z.B. Wasserstoffperoxid, Natriumperoxid, Bariumperoxid oder Peroxycarbonsäuren, insbesondere Peroxyameisensäure, Peroxyessig- und Peroxypropionsäure etc., die für den Fachmann in bekannter Weise auch in situ hergestellt bzw. erfindungsgemäß eingesetzt werden können. Die vorgenannten Verbindungen können allein aber auch als Mischung von wenigstens zwei dieser Verbindung im erfindungsgemäßen Verfahren Anwendung finden. Erfindungsgemäß wird das Bleichmittel in einer Menge 1,0 Gew.-% bis 10,0 Gew.-%, vorzugsweise 1,0 Gew.-% bis 3,0 Gew.-%, bezogen auf die gesamte Ansatzmenge, dem Bleichgut zugesetzt, wobei das bevorzugt einzusetzende Bleichmittel wäßrige Wasserstoffperoxidlösung ist.

Die Starttemperatur zur Behandlung der wässrigen Suspension des Mehles aus natürlichen Kernen, Schalen, Fruchthülsen und/oder Samen liegt bei 20 bis 40°C, vorzugsweise 25 bis 35°C und besonders bevorzugt 28 bis 32°C.

Für die vorliegende Erfindung ist es wesentlich, daß die Zugabe des Bleichmittels in zwei Schritten erfolgt, wobei im ersten Schritt die Entkeimung des Mehles im sauren Medium zu Beginn des Bleichprozesses nach Zugabe von wenigstens 50 bis 90 Gew.-% des Bleichmittels, vorzugsweise bis 80 Gew.-%, besonders bevorzugt 50 bis 70 Gew.-%, bezogen auf die gesamte Einsatzmenge des Bleichmittels, erfolgt. Der pH-Wert des Mediums beträgt hierbei insbesondere 3 bis 5, vorzugsweise 4 bis 5.

Die anschließende Zugabe der Bleichmittelrestmenge im zweiten Schritt zur Aufhellung und Entfettung des Bleichgutes erfolgt im alkalischen Milieu simultan mit einer Alkalienlösung. Insbesondere 10 bis 50 Gew.-%, vorzugsweise wenigstens 15 Gew.%, besonders bevorzugt 30 bis 50 Gew.-% der Bleichmittelrestmenge werden mit einer Alkalienlösung simultan zudosiert, wobei die Aufhellung und Entfettung des Bleichgutes bei einem pH-Wert von 7,0 bis 11,0, vorzugsweise 7,3 bis 9,0, besonders bevorzugt 7,8 bis 8,5 bewirkt wird.

Die Zugabe des Bleichmittels zum Bleichgut erfolgt in beiden Schritten aus Gründen der besseren Dosierung bzw. der pH-Wert-Steuerung vorzugsweise in flüssiger Form. Diese Zugabeform des Bleichmittels ist hierauf jedoch nicht beschränkt, sondern ist abhängig von der Wahl des Bleichgutes bzw. des einzusetzenden Bleichmittels. So ist es erfindungsgemäß auch möglich, die Zugabe des Bleichmittels zunächst in fester Form zur Bleichgutsuspension zu geben und im zweiten Schritt die Bleichmittelrestmenge in flüssiger Form simultan mit der Alkalienlösung zuzudosieren.

Da es sich bei dem erfindungsgemäßen Verfahren um einen industriellen Großprozeß handelt, ist aus Gründen der Wirtschaftlichkeit Wasser das besonders bevorzugte Lösungsmittel des benötigten Bleichmittels. Nichtsdestoweniger kann es auch hier im Hinblick auf die Art des Bleichgutes bzw. des benötigten Bleichmittels angezeigt sein, das Bleichmittel in einem üblichen organischen Lösungsmittel zu lösen, um die gewünschte Bleichwirkung zu erzielen. Die erhaltene Bleichmittellösung wird dann, wie beschriebenen, in zwei Schritten der Suspension des Bleichgutes zugesetzt.

Als Alkalienlösung werden üblicherweise wässrige Lösungen von Hydroxiden der Alkalimetalle, insbesondere von Natrium- und Kaliumhydroxid eingesetzt. Weiterhin sind auch Ammoniumhydroxid und die Hydroxide der Erdalkalimetalle sowie auch die Carbonate der Alkalimetalle, insbesondere Natriumcarbonat und Kaliumcarbonat verwendbar. Besonders bevorzugt ist jedoch wässrige Natriumhydroxidlösung bzw. Natronlauge als erfindungsgemäß einzusetzende Alkalienlösung, insbesondere als 35 bis 75 %-ige, vorzugsweise 45 bis 60 %-ige und besonders bevorzugt als 50 %-ige wässrige Lösung.

Die Verfahrensdauer des erfindungsgemäßen Verfahrens beträgt 160 bis 320 Minuten, vorzugsweise 180 bis 220 Minuten und besonders bevorzugt 190 bis 210 Minuten.

Aufgrund der schonenden Verfahrensführung liegt die Endtemperatur der natürlichen Kern-, Schalen-, Fruchthülsen- und/oder Samenmehl-Suspension nach der Bleichmittelbehandlung, insbesondere mit Wasserstoffperoxid-Lösung lediglich ca. 15 bis 20°C über der Starttemperatur.

Die Bleichsuspension wird dann nach üblichen bekannten Verfahren entwässert und das feuchte Mehl mit heißem Wasser von 70 bis 95°C, bevorzugt 80 bis 93°C und besonders bevorzugt 85 bis 92°C gewaschen und anschließend einem intensiven Trocknungsprozess unterzogen. Das getrocknete Mehl wird in Großgebinden abgefüllt und seinem Einsatz als Abrasivum in kosmetischen Präparaten beispielsweise in lösungsmittelfreien oder lösungsmittelhaltigen Handwaschpasten, in wasserfreien Hautreinigungsmitteln und in Peeling-Cremes direkt zugeführt.

Die Vorteile des erfindungsgemäßen Verfahrens sind im einzelnen:
Die durch das erfindungsgemäße Verfahren erhaltenen Verfahrensprodukte weisen nicht nur eine vergleichbare, sondern zum Teil sogar verbesserte Produktqualität zu den im Stand der Technik bekannten Abrasiva auf, insbesondere im Hinblick auf die Helligkeit der Produkte.

So konnte mittels physikalischer Farbmessung mit einem Farbmeßgerät nach DIN 5033 gefunden werden, daß die erfindungsgemäßen Verfahrensendprodukte einen L*-Wert von mindestens 81 aufweisen.

Die Werteskala der Werte, die der L*-Wert annehmen kann, erstreckt sich von 0 für ideal schwarze Farben bis 100 für Ideal-Weiß (siehe DIN 5033 sowie dort zitierte DIN-Normen; vgl. auch DIN 6174).

Hierdurch konnte die Farbaufhellung effektiver gestaltet werden, was für den Einsatz in kosmetischen Produkten im Hinblick auf die Optik des kosmetischen Endproduktes vorteilhaft ist.

Die nach dem erfindungsgemäßen Verfahren gebleichten Naturprodukte weisen im groß- bzw. produktionstechnischen Maßstab lediglich eine Ausschußquote durch Ver-keimungen unter 0,5 %, d.h. von maximal 0,2% auf, wodurch im Vergleich zu dem in der DE 103 05 959 beschriebenen Verfahren sich dieser großtechnische Bleichprozeß hinsichtlich der Logistik bezüglich Lagerhaltung, Transport und Mischungsprozesse enorm vereinfacht und somit betriebswirtschaftlich kostengünstiger gestaltet. Darüberhinaus weisen die erfindungsgemäßen Abrasiva eine sehr gute mikrobiologische Stabilität auf, wobei eine gleichmäßige Aufhellung/Farbe bei möglichst geringem Fettgehalt über die gesamte Bleichkampagne sichergestellt ist.

Die Entfettung verläuft gegenüber den im Stand der Technik bekannten Verfahren vollständiger, so daß Auswirkungen und damit Qualitätsunterschiede auf die Viskosität der kosmetischen Endprodukte ausgeschlossen werden kann.

Mit Hilfe des Beispiels 1 wird das Verfahren zur Herstellung des erfindungsgemäßen abrasiven Stoffes näher erläutert.

### Beispiel 1

### Bleichprozeß von natürlichem Schalen- oder Kernmehl mit Wasserstoffperoxid

Der Bleichprozess wird in einem 1.000 Liter-Reaktionsbehälter aus Edelstahl mit einem Intensivrührwerk und integrierter Doppel-pH-Wert- sowie Temperaturmessung durchgeführt.

Die Ansatzgrößen betragen zwischen 419,0 bis 450,8 kg.

### Rezeptur zu Beispiel 1

| **Rohstoff** | **Einsatzmenge/kg** |
|---|---|
| Wasser | 215 bis 220 |
| Schalen- oder Kernmehl Korngröße kleiner 200µm | 100 bis 105 |
| Wasserstoffperoxid 35%ig | 7,3 bis 7,6 |
| Natronlauge 50%ig | 2,2 bis 2,3 |
| Wasser zum Waschen | 135 bis 150 |

Das Wasser wird in dem Reaktionsbehälter vorgelegt und die o.g. Einsatzmenge an Schalen- oder Kernmehl unter Rühren eingetragen. Zu dieser Suspension werden Zweidrittel der Bleichmittelmenge an Wasserstoffperoxidlösung zugegeben und bei einem pH-Wert von 4 und 5 wird der Entkeimungsprozeß durchgeführt.

Anschließend erfolgt die Simultandosierung der Bleichmittelrestmenge an Wasserstoffperoxidlösung mit 50%iger Natronlaugenlösung im alkalischen Milieu bei einem pH-Wert von 7 bis 10.

Nach 160 bis 230 Minuten ist der Bleichprozeß abgeschlossen, wobei die Temperatur des fertiggebleichten Ansatzes um ca. 15°C über der Starttemperatur des Rohansatzes liegt.

Die erhaltene Bleichmittelsuspension wird entwässert, das gebleichte Mehl kontinuierlich wird gewaschen, getrocknet, in Großgebinde abgefüllt und direkt seiner weiteren Verarbeitung in kosmetischen Präparaten zugeführt.

In den Beispielen 2 und 4 werden eine lösungsmittelfreie und eine lösungsmittelhaltige erfindungsgemäße Formulierung einer Handwaschpaste und eines wasserfreien Hautreinigungsmittels mit gebleichtem Schalen- und/oder Kernmehl angegeben.

### Beispiel 2

**Formulierung einer lösungsmittelfreien Handwaschpaste**

| **Rohstoff** | **Einsatzmenge/Gew.-%** |
|---|---|
| Tensidkombination bestehend aus Natriumlaurylethersulfat 26%ig und Laurylalkoholpolyglycolether | 19,0 |
| Rizinusölsulfonat 68%ig | 5,1 |
| Raffiniertes Rapssamenöl | 9,0 |
| Wasser | 47,4 |
| Carboxymethylcellulose | 0,7 |
| Heteropolysaccharid, z.B. Xanthan Gum | 0,3 |
| Gebleichtes Schalen- oder Kernmehl | 14,0 |
| Olein | 1,2 |
| Titandioxid | 0,5 |
| Zitronensäure | 0,3 |
| Natriumchlorid | 1,5 |
| Konservierungsmittel | 0,8 |
| Parfüm | 0,2 |

### Beispiel 3

**Formulierung einer lösungsmittelhaltigen Handwaschpaste**

| **Rohstoff** | **Einsatzmenge/Gew.-%** |
|---|---|
| Tensidkombination bestehend aus Natriumlaurylethersulfat 28%ig und Cocoamidopropylbetain 30%ig | 52,0 |
| Rizinusölsulfonat 68%ig | 10,0 |
| Wasser | 0,8 |
| Carboxymethylcellulose | 0,5 |
| Organophiler Bentonit | 2,1 |
| Tetra-n-Butan (C₁₂-C₁₆-Alkane) Handelsprodukt der Fa. Oxeno | 17,0 |
| Gebleichtes Olivenkernmehl | 13,0 |
| Siedesalz | 2,8 |
| Zitronensäure | 0,3 |
| Titandioxid | 0,5 |
| Konservierungsmittel | 0,8 |
| Parfüm | 0,2 |

### Beispiel 4

**Formulierung eines wasserfreien Hautreinigungsmittels**

| **Rohstoff** | **Einsatzmenge/Gew.-%** |
|---|---|
| Tensid: Fettalkohol C12-C18, 5EO | 19,5 |
| Lösungsmittel bestehend aus Dimethyladipat, -glutarat, -succinat | 49,5 |
| Vernetztes Polyacrylsäure-Natriumsalz | 4,0 |
| Celluloseacetobutyrat | 3,5 |
| Isooctylstearat | 3,9 |
| Gebleichtes Walnußschalenmehl | 13,0 |
| Organophiler Bentonit | 4,4 |
| Propylencarbonat | 0,6 |
| Titandioxid | 1,0 |
| Pyrogene Kieselsäure | 0,3 |
| Parfüm | 0,3 |

Die Herstellung der Produkte erfolgt nach den üblichen bekannten Verfahren, die allgemein für die Formulierung von Tensid-Systemen bekannt sind. (G. Ziolkowski, Kosmetik-Jahrbuch 1986, 1987, 1989, Verlag für Chemische Industrie, H. Ziolkowski KG, Augsburg, Kosmetik Georg-Thieme-Verlag Stuttgart).

Die nach Beispiel 1 erhältlichen Chargen von gebleichten Naturmehlen wurden über einen Zeitraum von 24 Monaten untersucht, wobei 1100 Proben zur mikrobiologischen Bestimmung des Keimgehaltes bzw. der Feststellung auf Abwesenheit von pathogenen Keimen gezogen wurden.

Die Ergebnisse wiesen weitaus weniger verkeimte Muster aus den geprüften big bags auf als es bei dem Verfahren gemäß der DE 103 05 959 der Fall war. Während gemäß dem Verfahren nach der DE 103 05 959 ca. 15 % der gebleichten Ware wegen Verkeimung wieder in den Produktionsprozeß zurückgeführt werden mußte, sind es jetzt nach dem erfindungsgemäßen Verfahren lediglich ca, 0,2 %. Darüberhinaus beträgt die Kosteneinsparung des erfindungsgemäßen Herstellungsprozesses gegenüber dem vorhergehenden Verfahren ca. 30 %.

### Physikalische Farbmessung des Helligkeitsgrades der Verfahrensprodukte (L*-Wert) mit einem Farbmeßgerät nach DIN 5033

Zur Messung des Helligkeitsgrades wurde als Meßprinzip die Farb- und Farbdifferenzmessung nach dem 3-Bereichsverfahren gemäß DIN 5033 verwendet.

Als Meßgerät hierfür diente das 3-Bereichs-Farbmeßgerät MIKRO COLOR II der Fa. Dr. Lange Bruno Lange GmbH Berlin Industriemeßtechnik, Düsseldorf mit einem optischen Aufbau gemäß DIN 5033. Als Lichtquelle wurde eine Xenon-Blitz-Lampe verwendet, die in Verbindung mit einer Ulbricht schen Kugel zur diffusen Beleuchtung der zu messenden Probe dient - Normlichtart D65. Gemessen wird hierbei nach DIN 5033 die diffuse Reflexion der Probe unter einem Winkel von 8°.

Für die Farbmessung gemäß DIN 5033 wurde als Bezugsstandard bzw. als Weißstandard der nach DIN 55350 Teil 18, 4.1.2 zertifizierte Kalibrierstandard LZM 076 verwendet:

| | |
|---|---|
| Standardnummer: | 010799 |
| | |
| Normfarbwert X: | 74,5 |
| Normfarbwert Y: | 79,5 |
| Normfarbwert Z: | 83,0 |
| Normlichtart: | D65 |
| Normalbeobachter: | 10° |
| Meßgeometrie: | d/8° |

In der nachfolgenden Tabelle werden die L*-Werte verschiedener Chargen von erfindungsgemäß gebleichtem Walnußschalenmehl angegeben.

**Tabelle der Farbwerte:**

| **Verfahren nach** EP 0559 696 B1 | | |
|---|---|---|
| **Datum** | **Chargennummer** | **Farbe L*-Wert** |
| 01.12.1999 | 0040256813 | 70,5 |
| 02.12.1999 | 0040256816 | 70,9 |
| 06.12.1999 | 0040256817 | 71,4 |
| 16.12.2000 | 0040290639 | 74,3 |
| 28.03.2001 | 0040299877 | 72,3 |
| 11.09.2002 | 0040344064 | 73,8 |
| 26.04.2003 | 0040360168 | 74,7 |
| 18.05.2003 | 0040361975 | 71,9 |

| **erfindungsgemäßes Verfahren** | | |
|---|---|---|
| **Datum** | **Chargennummer** | **Farbe L*-Wert** |
| 09.10.2004 | 0040385914 | 81,8 |
| 17.10.2004 | 0040396622 | 82,3 |

### Physikalische Messung des Fettgehaltes

Die Prüfungen zur Entfettung der nach dem erfindungsgemäßen Verfahren erhaltenen Mehle erfolgte nach der DGF-Einheitsmethode B-1 5 (87). Diese Methode wird üblicherweise zur Bestimmung des Ölgehaltes von Ölsaaten, die als industrielle Ausgangsprodukte für die Herstellung von Fetten und Ölen verwendet werden, eingesetzt bzw. die Methode bildet die Grundlage für die Bewertung von Ölsaaten nach ihrem Ölgehalt.

Die nach dem erfindungsgemäßen Bleichverfahren erhaltenen Mehle lieferten im Vergleich zu den nach DE 103 05 959 bzw. erhaltenen Verfahrensprodukten folgende Werte nach der vorgenannten DGF-Einheitsmethode:

**Tabelle der Fettgehalte:**

| **Verfahren gemäß** EP0559 696B1 **bzw.** DE103 05 959 | | |
|---|---|---|
| **Datum** | **Chargennummer** | **Fettgehalt / %** |
| 29.06.2000 | 0040264186 | 0,08 |
| 01.07.2001 | 0040306700 | 0,12 |
| 23.02.2002 | 0040326688 | 0,08 |
| 18.05.2003 | 0040361975 | 0,10 |

| **erfindungsgemäßes Verfahren** | | |
|---|---|---|
| **Datum** | **Chargennummer** | **Festgehalt / %** |
| 17.12.2003 | 0040376537 | 0,05 |
| 03.09.2004 | 0040385945 | 0,05 |
| 03.10.2004 | 0040385908 | 0,05 |
| 14.10.2004 | 0040396619 | 0,04 |
| 17.10.2004 | 0040396622 | 0,05 |

Durch das erfindungsgemäße Verfahren weisen die gebleichten Mehle einen deutlich geringeren Fettgehalt auf, wodurch Auswirkungen auf die Qualität der kosmetischen Präparate, die diese Mehle als Abrasiva enthalten, ausgeschlossen werden können.

Die Entfettung verlief gegenüber den Verfahren gemäß EP 559 696 B1 sowie DE 103 05 959 vollständiger, so daß Auswirkungen und damit Qualitätsunterschiede auf die Viskosität der Endprodukte weitestgehend ausgeschlossen werden konnten.

## Patentansprüche

1. Verfahren zur Herstellung eines abrasiven Stoffes, wobei natürliche Kerne, Schalen, Fruchthülsen und/oder Samen zu einem Mehl definierter Korngröße gemahlen werden, das Mehl in wässriger Suspension mit mindestens einem Bleichmittel behandelt wird, **dadurch gekennzeichnet, daß** die Zugabe des Bleichmittels in zwei Schritten erfolgt, wobei im ersten Schritt wenigstens 50 Gew.-% bis 90 Gew.-% der Bleichmittelgesamtmenge zugegeben und das Mehl im saurem Medium entkeimt wird und im zweiten Schritt die Aufhellung und Entfettung des Mehles durch Simultandosierung der Bleichmittelrestmenge mit einer Alkalienlösung in alkalischem Medium bewirkt wird.

2. Verfahren nach Anspruch 1, **dadurch gekennzeichnet, daß** mindestens ein Bleichmittel in einer Menge 1,0 bis 10,0 Gew.-%, bezogen auf die gesamte Ansatzmenge, dem Bleichgut zugesetzt wird.

3. Verfahren nach einem der Ansprüche 1 oder 2, **dadurch gekennzeichnet, daß** wäßrige Wasserstoffperoxidlösung als Bleichmittel verwendet wird.

4. Verfahren einem der Ansprüche 1 bis 3, **dadurch gekennzeichnet, daß** die Entkeimung im sauren Medium bei einem pH-Wert von 3 bis 5 erfolgt.

5. Verfahren nach einem der Ansprüche 1 bis 4, **dadurch gekennzeichnet, daß** die Aufhellung und Entfettung des Mehls bei einem pH-Wert von 7 bis 11 bewirkt wird.

6. Verfahren nach einem der Ansprüche 1 bis 5, **dadurch gekennzeichnet, daß** die Bleichmittelrestmenge 50 bis 10 Gew.% beträgt.

7. Verfahren nach einem der Ansprüche 1 bis 6, **dadurch gekennzeichnet, daß** als natürliche Kern-, Schalen-, Fruchthülsen- und/oder Samenmehle Walnuß-, Haselnuß-, Mandelschalenmehl, Oliven-, Aprikosen-, Pfirsich-, Kirsch- oder Pflaumenkernmehl, Mehle von Palmkernen und Kokosnüssen, Jojobafrüchten, Macademia-Nüssen, Pistazien- und Pinienschalen, Maiskolbenmehl, Weizenkleie, Hafermehl oder Holzmehle sowie beliebige Gemische derselben mit einer Korngröße von 50 bis 2000 µm verwendet werden.

8. Verfahren nach den Ansprüchen 1 bis 7, **dadurch gekennzeichnet, daß** die Endtemperatur der natürlichen Kern-, Schalen-, Fruchthülsen und/oder Samenmehl-. Suspension nach der Bleichmittelbehandlung im wesentlichen 15 bis 20°C über der Starttemperatur der Bleichbehandlung liegt.

## Claims

1. Method for producing an abrasive material, wherein natural cores, husks, fruit rinds and/or seeds are ground to form a flour of a defined particle size, the flour is processed in aqueous suspension with at least one bleaching agent, **characterised in that** the addition of the bleaching agent is carried out in two steps, at least from 50% by weight to 90% by weight of the entire quantity of bleaching agent being added and the flour being sterilised in the acidic medium in the first step and the lightening and degreasing of the flour being carried out in the second step by means of simultaneous metering of the residual quantity of bleaching agent with a solution of alkalis in an alkaline medium.

2. Method according to claim 1, **characterised in that** at least one bleaching agent is added to the goods to be bleached in a quantity of from 1.0 to 10.0% by weight, with respect to the entire starting quantity.

3. Method according to either claim 1 or claim 2, **characterised in that** aqueous hydrogen peroxide solution is used as a bleaching agent.

4. Method according to any one of claims 1 to 3, **characterised in that** the sterilisation is carried out in acid medium at a pH value of from 3 to 5.

5. Method according to any one of claims 1 to 4, **characterised in that** the lightening and degreasing of the flour is carried out at a pH value of from 7 to 11.

6. Method according to any one of claims 1 to 5, **characterised in that** the residual quantity of bleaching agent is from 50 to 10% by weight.

7. Method according to any one of claims 1 to 6, **characterised in that** walnut, hazelnut, almond shell flour, olive, apricot, peach, cherry or plum stone flour, flours of palm kernels and coconuts, jojoba fruits, macadamia nuts, pistachios and stone pine shells, maize meal, wheat brans, oatmeal or wood flours and any admixtures thereof with a grain size of from 50 to 2000 µm are used as natural core, husk, fruit rind and/or seed flours.

8. Method according to claims 1 to 7, **characterised in that** the final temperature of the natural core, husk, fruit rind and/or seed flour suspension after the bleaching agent processing is substantially from 15 to 20°C above the starting temperature of the bleaching processing operation.

## Revendications

1. Procédé de fabrication d'une matière abrasive, dans lequel on moud des noyaux naturels, des coquilles, des cosses et/ou des semences en une farine de granulométrie définie, on traite la farine en suspension aqueuse avec au moins un agent de blanchiment, **caractérisé en ce que** l'on opère l'ajout de l'agent de blanchiment en deux étapes, dans lesquelles on ajoute dans la première étape au moins 50 % en poids à 90 % en poids de la quantité totale d'agent de blanchiment et on stérilise la farine en milieu acide et on procède dans la deuxième étape à l'éclaircissage et au dégraissage de la farine par dosage simultané de la quantité restante d'agent de blanchiment avec une solution alcaline en milieu alcalin.

2. Procédé selon la revendication 1, **caractérisé en ce que** l'on ajoute au produit à blanchir au moins un agent de blanchiment en une quantité de 1,0 à 10,0 % en poids, rapportée à la quantité totale ajoutée.

3. Procédé selon l'une des revendications 1 ou 2, **caractérisé en ce que** l'on utilise comme agent de blanchiment une solution aqueuse de peroxyde d'hydrogène.

4. Procédé selon l'une quelconque des revendications 1 à 3, **caractérisé en ce que** l'on effectue la stérilisation en milieu acide à une valeur de pH de 3 à 5.

5. Procédé selon l'une quelconque des revendications 1 à 4, **caractérisé en ce que** l'on procède à l'éclaircissage et au dégraissage de la farine à une valeur de pH de 7 à 11.

6. Procédé selon l'une quelconque des revendications 1 à 5, **caractérisé en ce que** la quantité restante d'agent de blanchiment vaut 50 à 10 % en poids.

7. Procédé selon l'une quelconque des revendications 1 à 6, **caractérisé en ce que** l'on utilise comme farines de noyaux naturels, de coquilles, de cosses et/ou de semences, une farine de coquilles de noix, de noisettes, d'amandes, une farine de noyaux d'olives, d'abricots, de pêches, de cerises ou de prunes, des farines de noyaux de palmier et de noix de coco, de graines de jojoba, de noix de Macadam, de coques de pistaches et de pins parasols, une farine d'épis de maïs, du son de froment, une farine d'avoine ou une farine de bois, ainsi que des mélanges quelconques de ceux-ci avec une taille de grains de 50 à 2000 µm.

8. Procédé selon les revendications 1 à 7, **caractérisé en ce que** la température finale de la suspension de farine de noyaux naturels, de coquilles, de cosses et/ou de semences après le traitement de blanchiment se situe essentiellement 15 à 20°C au-dessus de la température initiale du traitement de blanchiment.
